# EUROPEAN PATENT SPECIFICATION

(11) **EP 4 051 212 B1**
(45) Date of publication and mention of the grant of the patent: **26.11.2025**
(21) Application number: 20811717.6
(22) Date of filing: 26.10.2020
(51) Int. Cl.: A61K 8/02, A23G 4/06, A23G 3/36, A23G 3/34, A61Q 11/00, A61K 8/9706, A61K 8/49, A61K 8/46, A23G 3/20, A23G 3/42, A23G 3/48

(54) **CHEWING GUM PRODUCT FOR INSTANT OPTICAL TOOTH WHITENING**
KAUGUMMI FÜR SOFORTIGE OPTISCHE ZAHNBLEICHUNG
CHEWING GUM PERMETTANT LE BLANCHIMENT INSTANTANÉ DES DENTS PAR EFFET D'OPTIQUE

(30) Priority: 29.10.2019 IT 201900019986
(43) Date of publication of application: 07.09.2022
(73) Proprietor: Perfetti Van Melle S.p.A., 20020 Lainate (Milano) (IT)
(72) Inventor: SARRICA, Andrea, 20020 Lainate (MI) (IT); WALZL, Martin, 20020 Lainate (MI) (IT); DELEO, Maurizio, 20020 Lainate (MI) (IT); SALMOIRAGHI, Guglielmo, 20020 Lainate (MI) (IT); SIMONE, Livio, 20020 Lainate (MI) (IT); FUJIMOTO, Keiji, 20020 Lainate (MI) (IT); BALDI, Gianni, 20020 Lainate (MI) (IT)
(74) Representative: Bianchetti & Minoja with Trevisan & Cuonzo IPS SRL
(86) International application number: PCT/IB2020/060025
(87) International publication number: WO 2021/084401

(56) References cited:
- EP-A1- 3 223 785
- EP-A1- 3 538 223
- EP-B1- 3 071 170
- EP-B1- 3 223 784
- EP-B1- 3 223 785
- WO-A1-2018/080499
- WO-A1-99/33437
- DE-U1- 202008 015 637
- US-A1- 2006 104 922
- US-A1- 2018 116 929
- DATABASE GNPD [online] MINTEL; 3 December 2018 (2018-12-03), ANONYMOUS: "Peppermint Flavour Breath Refreshing Strips", XP055693544, retrieved from https://www.gnpd.com/sinatra/recordpage/6161593/ Database accession no. 6161593
- DATABASE GNPD [online] MINTEL; 8 January 2004 (2004-01-08), ANONYMOUS: "Totalcare Breath Strips", XP055693539, retrieved from https://www.gnpd.com/sinatra/recordpage/247231/ Database accession no. 247231
- DATABASE GNPD [online] MINTEL; 24 April 2017 (2017-04-24), ANONYMOUS: "Lemonade Flavour Sweets with Dextrose", XP055693614, retrieved from https://www.gnpd.com/sinatra/recordpage/4771931/ Database accession no. 4771931
- DATABASE GNPD [online] MINTEL; 16 January 2007 (2007-01-16), ANONYMOUS: "Fresh Breath Liquid Mints", XP055693547, retrieved from https://www.gnpd.com/sinatra/recordpage/637024/ Database accession no. 637024
- DATABASE GNPD [online] MINTEL; 8 January 2004 (2004-01-08), ANONYMOUS: "Totalcare Breath Strips", XP055693539, retrieved from www.gnpd.com Database accession no. 247231
- DATABASE GNPD [online] MINTEL; 3 December 2018 (2018-12-03), ANONYMOUS: "Peppermint Flavour Breath Refreshing Strips", XP055693544, retrieved from www.gnpd.com Database accession no. 6161593
- DATABASE GNPD [online] MINTEL; 24 April 2017 (2017-04-24), ANONYMOUS: "Lemonade Flavour Sweets with Dextrose", XP055693614, retrieved from www.gnpd.com Database accession no. 4771931

## Description

The invention relates to confectionery products for instant optical tooth whitening comprising or consisting of a fast-dissolving matrix containing at least one blue dye.

### PRIOR ART

Optical tooth whitening products which use the combination of dyes with a spectrum between violet and blue-violet are known. When applied, said products cause the teeth to reflect a whiter light than that which the teeth would have reflected naturally (US6030222).

The use of said dyes or mixtures thereof is also known in the field of confectionery products in general and chewing gum in particular, as described generically in US2006/0104922 together with other food products.

US2006/0104922 describes the use of dyes which have a peak absorption wavelength ranging from 480 to 660 nm. The food products described in US2006/0104922 must preferably remain in contact with the teeth for as long as possible in order to exercise the desired whitening effect. The chewing gum is designed to release the dye gradually during chewing. However, the prior art does not report any preferred formulations of confectionery products, nor any quantitation of the effect that said formulations can have.

EP3538223 A1 discloses a tooth whitening oral care composition comprising a blue dye and/or blue pigment such as Food blue 5 (E131) or FD&C blue 1 (E133). Various vehicles / formulation types are proposed, including lozenges or chewing gum.

Formulations containing dyes can have undesirable staining effects, for example on the tongue. The prior art does not identify those of the blue dyes which do not stain the tongue, or only stain it minimally, while still remaining effective in terms of instant optical whitening.

### DESCRIPTION OF THE INVENTION

It has now been found that it is possible to achieve optical tooth whitening without long-term consumption of a food product, by using confectionery products which contain suitable dyes and are formulated so that the dyes are rapidly made available after fast dissolution of the product, unlike the processes reported in the prior art.

In particular, instant optical tooth whitening can be obtained by consuming a confectionery product comprising a fast-dissolving matrix containing at least one blue dye selected from E131, E132, E133 and spirulina.

Suitable formulations comprise chewing gums with a candy coating layer containing said dyes.

"Fast-dissolving" means a time of less than 5 minutes, preferably less than 4 minutes. For example, a fast-dissolving confectionery preparation can dissolve in 3-4 minutes or in less than 30 seconds.

Dyes suitable for the purposes of the invention comprise Patented Blue V, indigotine, Brilliant Blue FCF and spirulina. Patented Blue V, indigotine and Brilliant Blue FCF are identified by the European food additives coding system as E131, E132 and E133 respectively. The dyes indigotine (E132) and spirulina are preferred, the latter with an intensity index of 1400 ± 140 for gums or 135-165 for tablets.

Spirulina is a natural dye derived from seaweed, and is standardised to the values listed in the intensity index (dyeing strength). The use of reducing-sugar-free carriers in solid form, such as maltodextrins, starches or gum arabic, is preferred, as they enable the dyeing intensity of spirulina to be increased to over 1000, and in a particularly preferred way to over 1200.

If spirulina is used in the candy coating of chewing gum, the use of a spirulina with an intensity index of 1400 ± 140, in an amount ranging from 0.1 to 100 mg per piece of gum, is preferred. The even more preferred amount ranges from 0.4 to 20 mg, and in particular from 0.6 to 5 mg.

In the case of chewing gum with a candy coating, the amount of E132, E131 or E133 dye present in said coating generally ranges from 0.15 mg to 0.85 mg. In particular, the matrix is a candy coating containing dye E132, E131 or E133 in an amount ranging from 0.15 to 0.85 mg, or spirulina dye with an intensity index of 1400 ± 140 in an amount ranging from 0.1 to 100 mg.

The candy coating preferably comprises one or more polyols selected from xylitol, isomalt, maltitol, sorbitol and erythritol.

The confectionery products according to the invention can be obtained by conventional methods.

Methods have been found which are able to quantify the optical whitening effect by applying them for the first time to food products, to test the advantageous effect of specific formulations.
intensity of spirulina It has also been found that some dyes have an immediate optical whitening effect without appreciably staining the tongue.

"Optical whitening" means the deposit on the teeth of a minimum amount of dye which causes the colour of the tooth to be globally perceived as whiter.

An index known as the WIO has been developed to evaluate the degree of tooth whiteness. Said index is calculated according to the equation published by Luo et al., Development of a whiteness index for dentistry. Journal of dentistry 37s (2009) e21-e26. By measuring the colorimetric parameters before and after the application according to the invention, the ΔWIO can be calculated. The higher the ΔWIO, the more effective the optical whitening.

"Immediate" means an effect recorded after a few minutes' consumption of the product according to the invention, for example after 2.5, 5 or 10 minutes' chewing or consumption of a single piece of confectionery. Said times are much shorter than those generally used to test the effect of the non-optical type of whitening toothpastes, which typically amount to 6 weeks (ADA - Tooth Stain Removal Products - January 2019).

The confectionery products according to the invention present the advantage of a more intense, faster whitening effect than that of the formulations described in US 2006/0104922. Moreover, the confectionery products according to the invention do not cause undesirable staining of the consumer's tongue. The use of the dye indigotine or E132 gives the end product particular stability to fading caused by exposure of the packaged product to light. In fact, confectionery products are very often offered to the public in packaging characterised by "windows" consisting of transparent portions of packaging that enable the consumer to see the product inside it.

While on the one hand said transparent windows allow the product to be viewed and are more appealing to consumers, on the other they expose the product to light, and such exposure can cause the pigmentation of the product to break down, leading to fading of the dye. This can have exactly the opposite effect to that intended by using a transparent window, as the colour of the product in the packaging may fade, in particular in an uneven way, thus making it unattractive and giving the impression of an old, worn, or even defective product. This would directly affect the probability of consumers deciding to buy the product after viewing it.

The loss of colour would also reduce the efficacy of the optical whitening effect.

The above-mentioned advantageous effects are demonstrated in the tests described below.

### EXAMPLE 1 - Comparison between chewing gum formulations that differ solely in terms of whether the dye is positioned in the coating or core of the product.

The chewing gums tested were characterised by "Formula 1" as regards the core and "Formula 2" as regards the coating:

### Formula 1

| **Ingredients** | **%** |
|---|---|
| Gum base | 35,00 |
| Maltitol syrup | 4,00 |
| Sorbitol | 36,00 |
| Mannitol | 10,00 |
| Xylitol | 7,00 |
| Spray dried flavour | 4,00 |
| Aspartame | 0,50 |
| Acesulfame K | 0,25 |
| Sucralose | 0,25 |
| Glycerol | 1,00 |
| Liquid flavour | 2,00 |
| **Total** | **100,00** |

### Formula 2

| **Ingredients** | **%** |
|---|---|
| Isomalt | 96,00 |
| Maltodextrin | 1,00 |
| Sucrose esters | 0,30 |
| Aspartame | 0,20 |
| Acesulfame K | 0,20 |
| Flavour | 2,20 |
| Carnauba wax | 0,10 |
| **Total** | **100,00** |

The dyes listed in Table 1 below were added to the chewing gums thus formulated, in the core or the coating, in the amounts stated:

**Table 1**

| **Test no.** | **3 - placebo gum** | | **4 - gum with E131 core** | | **5 - gum with E131 coating** | | **6 - gum with E132 core** | | **7 - gum with E132 coating** | | **8 - gum with E133 core** | | **9 - gum with E133 coating** | |
|---|---|---|---|---|---|---|---|---|---|---|---|---|---|---|
| **Ingredients** | **Mg** | **%** | **mg** | **%** | **mg** | **%** | **mg** | **%** | **mg** | **%** | **mg** | **%** | **mg** | **%** |
| Core test 1 | 1050.00 | 75.00 | 1049.70 | 74.98 | 1050.00 | 75.00 | 1049.70 | 74.98 | 1050.00 | 75.00 | 1049.70 | 74.98 | 1050.00 | 75.00 |
| Coating test 2 | 350.00 | 25.00 | 350.00 | 25.00 | 349.70 | 24.98 | 350.00 | 25.00 | 349.70 | 24.98 | 350.00 | 25.00 | 349.70 | 24.98 |
| E131 in core | | | 0.30 | 0.02 | | | | | | | | | | |
| E131 in coating | | | | | 0.30 | 0.02 | | | | | | | | |
| E132 in core | | | | | | | 0.30 | 0.02 | | | | | | |
| E132 in coating | | | | | | | | | 0.30 | 0.02 | | | | |
| E133 in core | | | | | | | | | | | 0.30 | 0.02 | | |
| E133 in coating | | | | | | | | | | | | | 0.30 | 0.02 |
| Total | 1400.00 | 100.00 | 1400.00 | 100.00 | 1400.00 | 100.00 | 1400.00 | 100.00 | 1400.00 | 100.00 | 1400.00 | 100.00 | 1400.00 | 100.00 |

The whitening effect on test participants was measured with the VitaEasyshade Advance 4.0 instrument. The six participants who took part in the test chewed 1 piece of chewing gum for 5 minutes in the case of examples 3, 4, 5, 8 and 9, and 2 pieces of chewing gum for 10 minutes in the case of examples 6 and 7.

Each measurement was conducted on the front teeth in triplicate, giving the colorimetric parameters according to the CIELAB system, which were then combined according to the equation published by Luo et al. "Development of a whiteness index for dentistry", Journal of Dentistry 37s (2009) e21-e26, to determine the mean whiteness index (WIO) before and after chewing, and therefore its variation ΔWIO.

The results are set out in Table 2 below.

**Table 2**

| **Sample** | **Δ WIO** |
|---|---|
| Experiment 3 - Placebo gum | 0.09 |
| Experiment 4 - Gum E131 CENTER | 1.44 |
| Experiment 8 - Gum E133 CENTER | 1.37 |
| Experiment 6 - Gum E132 CENTER | 2.40 |
| Experiment 5- Gum E131 COATING | 4.45 |
| Experiment 9 - Gum E133 COATING | 6.34 |
| Experiment 7 - Gum E132 COATING | 3.60 |

### EXAMPLE 2 - Tongue staining evaluation

Tongue staining was evaluated with a test wherein participants were asked to chew various product samples, as listed below:

| **Sample** |
|---|
| Experiment 5 - Gum with E131 COATING |
| Experiment 9 - Gum with E133 COATING |
| Experiment 7 - Gum with E132 COATING |
| Experiment 13 - Gum with spirulina COATING |

The sample of chewing gum containing spirulina was made as indicated below:

| **Experiment no. 13 - Gum with spirulina coating** | | |
|---|---|---|
| **Ingredients** | **Mg** | **%** |
| Core Formula 1 | 1050.00 | 75.00 |
| COATING Formula 2 | 349.00 | 24.93 |
| Spirulina inserted in coating | 1.00 | 0.07 |
| Total | 1400.00 | 100.00 |

The samples were chewed for various time intervals, the participants' tongues were photographed at the end of each chewing interval, the photographs were compared, and the level of tongue staining was calculated by the assessor on the following scale:
0 = no staining.
1 = slight staining of part of the tongue, in particular only the edges or centre thereof.
2 = slight staining of 1/3 to 2/3 of the tongue.
3 = evident staining of part of the tongue, in particular only the edges or centre thereof.
4 = evident staining of 1/3 to 2/3 of tongue.

The results relating to the test samples chewed for the time intervals specified above are set out in Table 3 below for cycles characterised by chewing of a dragée for 15 seconds, followed by a 5-minute pause. The time intervals were:
Time 1= before chewing:
Time 2 = immediately after chewing of first dragée
Time 3 = after completion of 4 cycles (4 dragées chewed, one per cycle), and immediately before chewing of fifth-cycle dragée.
Time 4 = immediately after chewing of fifth dragée for 15 seconds.

**Table 3**

| **Sample** | **Time 1** | **Time 2** | **Time 3** | **Time 4** |
|---|---|---|---|---|
| Experiment 5 - Gum with E131 COATING | 0 | 4 (stop) | | |
| Experiment 9 - Gum with E133 COATING | 0 | 4 (stop) | | |
| Experiment 7 - Gum with E132 COATING | 0 | 1 | 0 | 1 |
| Experiment 13 - Gum with spirulina COATING | 0 | 0 | 0 | 0 |

The experiment was repeated for cycles characterised by chewing of a dragée for 2 minutes, followed by a 5-minute pause. The time intervals were as reported above. The results are set out in Table 4 below.

**Table 4**

| **Sample** | **Time 1** | **Time 2** | **Time 3** | **Time 4** |
|---|---|---|---|---|
| Experiment 5 - Gum with E131 COATING | 0 | 3 (stop) | | |
| Experiment 9 - Gum with E133 COATING | 0 | 3 (stop) | | |
| Experiment 7 - Gum with E132 COATING | 0 | 0,5 | 0 | 0,5 |
| Experiment 13 - Gum with spirulina COATING | 0 | 0 | 0 | 0 |

### EXAMPLE 4 - Stability to light

Stability to colour fading caused by exposure to light was evaluated with the SuntestXLS + instrument made by Atlas, which simulates exposure to sunlight for periods of 3, 6, 9 and 12 months.
The samples used for the test were as follows:

| Sample |
|---|
| Experiment 5 - Gum with E131 COATING |
| Experiment 9 - Gum with E133 COATING |
| Experiment 7 - Gum with E132 COATING |

The measurement was taken according to the CIELAB colorimetric model whereby a colour is identified by the three values "L" (lightness) and "a" and "b", two colour ranges that range from green to red and blue to yellow respectively, with values from -120 to +120.

The change in colour of the samples was calculated by measuring parameter ΔE. The results are set out in Table 5 below, which demonstrates that the example containing E132 exhibits a minimal colour change compared with the examples containing E131 and E133:

**Table 5**

| **ΔE to baseline** | | | |
|---|---|---|---|
| **Month** | **Experiment 5 (E131)** | **Experiment 7 (E132)** | **Experiment 9 (E133)** |
| **0** | 0.00 | 0.00 | 0.00 |
| **3** | 2.85 | 1.24 | 5.97 |
| **6** | 4.24 | 1.11 | 7.88 |
| **9** | 5.76 | 1.54 | 8.20 |
| **12** | 6.86 | 1.45 | 9.14 |

## Claims

1. A confectionery product in the form of chewing gum comprising a fast-dissolving matrix containing at least one blue dye selected from E131, E132, E133 and spirulina.

2. A confectionery product according to claim 1 wherein the dye is spirulina.

3. A confectionery product according to claim 1 wherein the dye is E132.

4. A confectionery product according to claim 1 in the form of a chewing gum wherein the matrix is a candy coating containing dye E132, E131 or E133 in an amount ranging from 0.15 to 0.85 mg, or spirulina dye with an intensity index of 1400 ± 140 in an amount ranging from 0.1 to 100 mg.

5. A confectionery product according to claim 4 wherein the candy coating comprises one or more polyols selected from xylitol, isomalt, maltitol, sorbitol and erythritol.

6. A confectionery product according to claim 1, 2 or 3 consisting of a matrix in the form of a fast-dissolving tablet.

7. A confectionery product according to claim 6 containing spirulina with an intensity index of 135-165 in an amount ranging from 0.1 mg to 10 mg.

8. A confectionery product according to claim 6 containing E132, E131 or E133 in an amount ranging from 0.10 mg to 0.30 mg.

9. A confectionery product according to claims 1 to 8 wherein the fast-dissolving matrix dissolves in a time of less than 5 minutes.

10. The use of a confectionery product according to claims 1-8 for the optical teeth whitening.

## Patentansprüche

1. Süßwarenprodukt in Form von Kaugummi, umfassend eine sich schnell auflösende Matrix, die mindestens einen blauen Farbstoff enthält, der ausgewählt ist aus E131, E132, E133 und Spirulina.

2. Süßwarenprodukt gemäß Anspruch 1, wobei der Farbstoff Spirulina ist.

3. Süßwarenprodukt gemäß Anspruch 1, wobei der Farbstoff E132 ist.

4. Süßwarenprodukt gemäß Anspruch 1 in Form eines Kaugummis, wobei die Matrix eine Süßwarenbeschichtung ist, die den Farbstoff E132, E131 oder E133 in einer Menge im Bereich von 0,15 bis 0,85 mg oder Spirulina-Farbstoff mit einem Intensitätsindex von 1400 ± 140 in einer Menge im Bereich von 0,1 bis 100 mg enthält.

5. Süßwarenprodukt gemäß Anspruch 4, wobei die Süßwarenbeschichtung ein oder mehrere Polyole umfasst, ausgewählt aus Xylit, Isomalt, Maltit, Sorbit und Erythrit.

6. Süßwarenprodukt gemäß Anspruch 1, 2 oder 3, bestehend aus einer Matrix in Form einer sich schnell auflösenden Tablette.

7. Süßwarenprodukt gemäß Anspruch 6, welches Spirulina mit einem Intensitätsindex von 135-165 in einer Menge im Bereich von 0,1 mg bis 10 mg enthält.

8. Süßwarenprodukt gemäß Anspruch 6, welches E132, E131 oder E133 in einer Menge im Bereich von 0,10 mg bis 0,30 mg enthält.

9. Süßwarenprodukt gemäß einem der Ansprüche 1-8, wobei sich die schnell lösliche Matrix in einer Zeit von weniger als 5 Minuten auflöst.

10. Verwendung eines Süßwarenprodukts nach einem der Ansprüche 1-8 zur optischen Zahnaufhellung.

## Revendications

1. Produit de confiserie sous forme de chewing gum comprenant une matrice à dissolution rapide contenant au moins un colorant bleu choisi parmi E131, E132, E133 et la spiruline.

2. Produit de confiserie selon la revendication 1, dans lequel le colorant est la spiruline.

3. Produit de confiserie selon la revendication 1, dans lequel le colorant est E132.

4. Produit de confiserie selon la revendication 1, sous forme de chewing gum, dans lequel la matrice est un enrobage sucré contenant du colorant E132, E131 ou E133 en une quantité allant de 0,15 à 0,85 mg, ou un colorant spiruline avec un indice d'intensité de 1400 ± 140 en une quantité allant de 0,1 à 100 mg.

5. Produit de confiserie selon la revendication 4, dans lequel l'enrobage sucré comprend un ou plusieurs polyols choisis parmi le xylitol, l'isomalt, le maltitol, le sorbitol et l'érythritol.

6. Produit de confiserie selon la revendication 1, 2 ou 3, constitué d'une matrice sous forme de comprimé à dissolution rapide.

7. Produit de confiserie selon la revendication 6 contenant de la spiruline ayant un indice d'intensité de 135 à 165, en une quantité allant de 0,1 mg à 10 mg.

8. Produit de confiserie selon la revendication 6 contenant E132, E131 ou E133 en une quantité allant de 0,10 mg à 0,30 mg.

9. Produit de confiserie selon les revendications 1 à 8, dans lequel la matrice à dissolution rapide se dissout en un temps inférieur à 5 minutes.

10. Utilisation d'un produit de confiserie selon les revendications 1 à 8 pour le blanchiment des dents par effet d'optique.
